# EUROPÄISCHE PATENTANMELDUNG

(11) **EP 4 759 241 A1**
(43) Veröffentlichungstag der Anmeldung: **17.06.2026**
(21) Anmeldenummer: 24220149.9
(22) Anmeldetag: 16.12.2024
(51) Int. Cl.: A61B 6/10

(54) **VERKLEIDUNGSANORDNUNG UND ABSCHALTEINRICHTUNG FÜR EIN MEDIZINISCHES GERÄT UND MEDIZINISCHES GERÄT**

(71) Anmelder: Siemens Healthineers AG, 91301 Forchheim (DE)
(72) Erfinder: Igney, Claudia, 91056 Erlangen (DE); Kleber, Thomas, 92709 Moosbach (DE); Lautner, Thomas, 95505 Immenreuth (DE); Schmidt, Verena, 92681 Erbendorf (DE); Schraml, Martin, 95506 Kastl (DE); Emmerig, Christoph, 95469 Speichersdorf (DE); Otlewski, Philipp, 95028 Hof (DE); Raschke, Daniel, 95447 Bayreuth (DE); Schmidt, Stefan, 92721 Störnstein (DE); Seitz, Marius, 92690 Pressath (DE); Wutz, Jörn, 95478 Kemnath (DE)
(74) Vertreter: Siemens Healthineers Patent Attorneys

(57) **Zusammenfassung**

Eine Verkleidungsanordnung (27) für ein medizinisches Gerät (1) weist eine erste Verkleidungskomponente (13) zur starren Befestigung an einer beweglichen Komponente (2) des medizinischen Geräts (1) auf, sowie eine zweite Verkleidungskomponente (14). Die erste Verkleidungskomponente (13) und die zweite Verkleidungskomponente (14) sind mittels einer elastischen Verbindungskomponente (15) miteinander verbunden und umschließen gemeinsam einen Innenraum (16). Die Verkleidungsanordnung (27) weist eine Kollisionserkennungsvorrichtung auf, die einen ersten taktilen Sensor (18) aufweist, der in dem Innenraum (16) angeordnet und dazu eingerichtet ist, bei einer ersten Bewegung eines Teils der zweiten Verkleidungskomponente (14) relativ zu der ersten Verkleidungskomponente (13) betätigt zu werden. Die Kollisionserkennungsvorrichtung weist eine Auswerteschaltung (28) auf, die dazu eingerichtet ist, ein erstes Warnsignal zu erzeugen, wenn der erste taktile Sensor (18) betätigt wurde.

## Beschreibung

Die vorliegende Erfindung betrifft eine Verkleidungsanordnung für ein medizinisches Gerät aufweisend eine erste Verkleidungskomponente zur starren Befestigung an einer beweglichen Komponente des medizinischen Geräts, sowie eine zweite Verkleidungskomponente. Die Erfindung betrifft ferner eine Abschalteinrichtung für ein medizinisches Gerät aufweisend eine solche Verkleidungsanordnung ein entsprechendes medizinisches Gerät.

Bewegliche Komponenten von medizinischen Geräten können sich teils mit hohen Geschwindigkeiten bewegen. Beispielsweise können einzelne Achsen von deckenmontierten Röntgenangiographiesystemen automatisierte Fahrten mit hohen Geschwindigkeiten realisieren, die für die Bildgebung von Bedeutung sind. Da solche Bewegungsabläufe potenziell Risiken für umstehende Personen und Gegenstände sowie für das medizinische Gerät selbst darstellen, ist es vorteilhaft, bei solchen medizinischen Geräten eine Abschalteinrichtung an der beweglichen Komponente oder mehrere Abschalteinrichtungen an verschiedenen Stellen der der beweglichen Komponente oder des medizinischen Geräts vorzusehen, welche die Bewegung der beweglichen Komponente bei Kollisionen der beweglichen Komponente mit anderen Gegenständen oder Personen deaktivieren können.

Zur Detektion der Kollision kann beispielsweise eine Schaltleiste außen an der beweglichen Komponente angebracht werden. Dies kann im Kontext medizinischer Geräte aus Hygienegründen unvorteilhaft sein beziehungsweise eine regelmäßige gründliche Reinigung der Schaltleiste und der Verbindungsstellen zu der beweglichen Komponente erfordern.

Es ist eine Aufgabe der vorliegenden Erfindung, eine einfache und robuste Möglichkeit zur Kollisionserkennung einer beweglichen Komponente eines medizinischen Geräts anzugeben, durch welche die genannten Nachteile überwunden werden können.

Dokument EP 4 389 012 A1 beschreibt eine zwischen einer Deckschicht und einer Grundschicht verlaufende Sensoreinheit umfassend zwei Sensorschichten, und eine zwischen den beiden Sensorschichten angeordnete, reversibel kompressible Abstandsschicht, wobei wenigstens zwei Schichten als mittels einer Technik der additiven Fertigung gebildete Schichten ausgebildet sind.

Diese Aufgabe wird durch den Gegenstand des unabhängigen Patentanspruchs gelöst. Vorteilhafte Weiterbildungen und bevorzugte Ausführungsformen sind Gegenstand der abhängigen Ansprüche.

Die Erfindung beruht auf der Idee, einen taktilen Sensor zur Kollisionserkennung vorzusehen, der in einem von zwei Verkleidungskomponenten umschlossenen Innenraum angeordnet ist und insbesondere bei einer bestimmten Bewegung oder Deformation der Verkleidungskomponenten relativ zueinander betätigt wird.

Gemäß einem Aspekt der Erfindung wird eine Verkleidungsanordnung für ein medizinisches Gerät angegeben. Die Verkleidungsanordnung weist eine erste Verkleidungskomponente zur starren Befestigung, insbesondere der ersten Verkleidungskomponente beziehungsweise damit auch der Verkleidungsanordnung, an einer beweglichen Komponente des medizinischen Geräts auf, sowie eine zweite Verkleidungskomponente. Die erste Verkleidungskomponente und die zweite Verkleidungskomponente sind mittels einer elastischen Verbindungskomponente miteinander verbunden und umschließen gemeinsam einen Innenraum, insbesondere einen Innenraum der Verkleidungsanordnung. Die Verkleidungsanordnung weist eine Kollisionserkennungsvorrichtung auf, die einen ersten taktilen Sensor aufweist, der in dem Innenraum angeordnet und dazu eingerichtet ist, bei einer ersten Bewegung eines Teils der zweiten Verkleidungskomponente relativ zu der ersten Verkleidungskomponente betätigt, insbesondere geschlossen, zu werden. Die Kollisionserkennungsvorrichtung weist eine Auswerteschaltung auf, die dazu eingerichtet ist, ein erstes Warnsignal zu erzeugen, wenn der erste taktile Sensor betätigt wurde oder wird.

Die erste Bewegung kann wenigstens zum Teil durch die elastische Verbindungskomponente ermöglicht werden. Die erste Bewegung kann beispielsweise eine Bewegung der gesamten zweiten Verkleidungskomponente sein. Die erste Bewegung kann aber auch zum Teil auf eine Verformung der zweiten Verkleidungskomponente zurückgehen. Die Position des taktilen Sensors beziehungsweise eines Auslösers oder Betätigungselements des taktilen Sensorsin dem Innenraum kann so gewählt sein, dass der taktilen Sensor dann betätigt wird, wenn die Bewegung des Teils der zweiten Verkleidungskomponente entlang einer oder mehrerer vordefinierter Richtungen und/oder mindestens über eine vordefinierte Mindeststrecke hinweg erfolgt. So können falsch positive Auslösungen vermieden werden.

Die Betätigung des taktilen Sensors kann beispielsweise direkt durch mechanische Einwirkung der zweiten Verkleidungskomponente auf den taktilen Sensor beziehungsweise den Auslöser oder das Betätigungselement erfolgen oder indirekt, indem die erste Bewegung der zweiten Verkleidungskomponente mittels eines oder mehrerer Übertragungselemente auf eine entsprechende Bewegung des Übertragungselements oder der Übertragungselemente übertragen wird und die Betätigung durch Einwirkung des Übertragungselements oder der Übertragungselemente auf den taktilen Sensorbeziehungsweise den Auslöser oder das Betätigungselement erfolgt.

Unter einem taktilen Sensor kann hier und im Folgenden ein Sensor verstanden werden, der dazu eingerichtet ist, eine mechanische Berührung oder eine mechanische Krafteinwirkung auf den Sensor zu detektieren, insbesondere abhängig von der mechanischen Berührung oder mechanischen Krafteinwirkung ein elektrisches Sensorsignal zu erzeugen.

Der erste taktile Sensor kann beispielsweise als Mikroschalter oder als flächiger Sensor wie in EP 4 389 012 A1 beschrieben ausgestaltet sein.

Die elastische Verbindungskomponente kann beispielsweise ein elastischer Klebstoff und/oder ein Elastomer und/oder ein Silikon sein. Die elastische Verbindungskomponente kann beispielsweise ein silanmodifiziertes Polymer, SMP, insbesondere ein SMP-Hybridpolymer und/oder ein SMP auf Polyetherbasis, oder ein Polyurethan-Klebstoff sein.

Die Integration des taktilen Sensors in den Innenraum verhindert den direkten Kontakt mit äußeren Einflüssen und erfordert keine Reinigung des taktilen Sensors oder von Verbindungsstellen, was zur hygienischeren Anwendung beiträgt. Die Verkleidungsanordnung schützt den taktilen Sensor vor Beschädigungen durch externe Einflüsse. Durch die robuste Konstruktion und den Schutz vor Umwelteinflüssen werden auch Wartungsaufwände minimiert.

Ein weiterer Vorteil der erfindungsgemäßen Verkleidungsanordnung folgt aus der Verbindung der Verkleidungskomponenten mittels der elastischen Verbindungskomponente. Dies erlaubt nämlich eine Bewegung der zweiten Verkleidungskomponente relativ zu der ersten Verkleidungskomponente unter einer Vielzahl von Randbedingungen, insbesondere für harte, unelastische Verkleidungskomponenten ebenso wie für weiche Verkleidungskomponenten oder eine Kombination einer harten mit einer weichen Verkleidungskomponente. Effektiv wirkt die elastischen Verbindungskomponente als Federelement welches die Verkleidungskomponenten miteinander koppelt. Auf diese Weise können gegebenenfalls anderweitig vorzusehende Federelemente eingespart werden.

Die beschriebene Verkleidungsanordnung kann in einer Vielzahl von medizinischen Geräten eingesetzt werden, darunter Röntgenbildgebungssysteme inklusive C-Arm Geräten, Röntgenangiographiesysteme, CT-Scanner, sowie MRT-Geräte, selbstfahrende medizinische Geräte oder Roboter für endovaskuläre oder sonstige chirurgische Eingriffe.

Die beanspruchte Verkleidungsanordnung bietet mehrere Vorteile. Zum einen ist sie einfach und preiswert in der Herstellung, da sie aus standardmäßigen Komponenten besteht. Zum anderen ist sie robust und kann eine regelmäßige Reinigung überstehen, was insbesondere im medizinischen Kontext von Bedeutung ist. Schließlich bietet sie einen hohen Standard an Hygiene, da die Kollisionserkennungsvorrichtung innerhalb des geschützten Innenraums untergebracht ist.

Die mögliche Verwendung der beanspruchten Verkleidungsanordnung beschränkt sich nicht nur auf medizinische Geräte. Sie kann in jeder Situation angewendet werden, wo bewegliche Komponenten Schutz vor Kollisionen benötigen. Beispiele hierfür sind automatisierte Transportsysteme, Robotik und ähnliche Anwendungen.

Die bewegliche Komponente kann beispielsweise beweglich bezüglich einer feststehenden, beispielsweise in einem Raum montierten, weiteren Komponente des medizinischen Geräts sein. Es ist auch möglich, dass das medizinische Gerät selbst mobil, beispielsweise selbstfahrend, ist. In diesem Fall kann jede Komponente des medizinischen Geräts als beweglich aufgefasst werden.

Der Innenraum wird jedenfalls zum Teil durch die erste Verkleidungskomponente und die zweite Verkleidungskomponente begrenzt. Dass die erste Verkleidungskomponente und die zweite Verkleidungskomponente gemeinsam einen Innenraum umschließen schließt insbesondere nicht aus, dass die erste Verkleidungskomponente und die zweite Verkleidungskomponente mittels einer oder mehrerer Verbindungskomponenten miteinander verbunden sind, sodass gegebenenfalls auch die eine oder die mehreren Verbindungskomponenten den Innenraum begrenzen.

Die Auswerteschaltung kann ein Datenverarbeitungssystem oder Teil eines Datenverarbeitungssystems, insbesondere der Verkleidungsanordnung, sein. Das Datenverarbeitungssystem und/oder die Auswerteschaltung kann vollständig oder teilweise in dem Innenraum angeordnet sein oder dazu ausgebildet sein außerhalb des Innenraums, beispielsweise an der Verkleidungsanordnung oder in oder an einer anderen Stelle des medizinischen Geräts, angeordnet zu werden.

In der vorliegenden Offenbarung können die Ausdrücke "Datenverarbeitungssystem" und "wenigstens ein Datenverarbeitungsgerät" austauschbar verwendet werden. Unter einem Datenverarbeitungsgerät kann insbesondere ein Datenverarbeitungsgerät verstanden werden, das einen Verarbeitungsschaltkreis enthält. Das Datenverarbeitungsgerät kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen ebenso wie ein in Hardware implementierter Datenverarbeitungsprozess.

Das Datenverarbeitungsgerät kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Das Datenverarbeitungsgerät kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere Digitalsignalprozessoren, DSP, enthalten. Das Datenverarbeitungsgerät kann auch einen physischen oder einen virtuellen Verbund von Computern oder sonstigen der genannten Einheiten beinhalten.

In verschiedenen Ausführungsbeispielen beinhaltet das Datenverarbeitungsgerät eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "readonly memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Gemäß zumindest einer Ausführungsform ist der erste taktile Sensor als Mikroschalter, insbesondere als erster Mikroschalter, ausgestaltet.

Ein Mikroschalter ist insbesondere ein elektrischer Schalter, dessen Kontakte im geöffneten Zustand weniger als 3 mm Abstand voneinander haben. Im Gegensatz zu anderen Schaltern wie Drucktastern oder Relais sind Mikroschalter besonders klein und können aufgrund ihrer geringen Baugröße und ihres niedrigen Schaltwegs in engen Räumen eingesetzt werden. Mikroschalter haben in der Regel eine sehr kurze Schaltzeit und einen geringen Schaltweg, was sie für Anwendungen geeignet macht, bei denen schnell auf Ereignisse reagiert werden muss oder wenig Platz vorhanden ist.

In Bezug auf die erfindungsgemäße Verkleidungsanordnung wird der Mikroschalter im Innenraum der Verkleidungsanordnung verwendet, um die erste Bewegung der zweiten Verkleidungskomponente zu erkennen. Durch seine geringe Größe und kurze Schaltzeit kann er schnell auf Bewegungen oder Kollisionen reagieren und so eine effektive Kollisionserkennung ermöglichen. Durch den Einsatz des Mikroschalters wird eine besonders zuverlässige und präzise Erfassung von Kollisionen selbst bei geringfügigen Deformationen der zweiten Verkleidungskomponente ermöglicht. Das Warnsignal signalisiert potenzielle Kollisionen und ermöglicht die sofortige, manuelle oder automatische, Abschaltung der beweglichen Komponente, um Schäden zu vermeiden.

Gemäß zumindest einer Ausführungsform ist der erste taktile Sensor, insbesondere der Mikroschalter, starr an der ersten Verkleidungskomponente befestigt.

Dadurch wird eine besonders präzise und zuverlässige Kollisionserkennung ermöglicht. Durch die starre Verbindung des taktilen Sensors mit der beweglichen Komponente wird sichergestellt, dass er jede relevante Bewegung oder Deformation der beweglichen Komponente erkennt.

Die starre Befestigung des taktilen Sensors an der ersten Verkleidungskomponente hat den Vorteil, dass der taktile Sensor immer in der gleichen Position bleibt und daher zuverlässig auf Betätigungen reagieren kann. Diese Anordnung gewährleistet eine hohe Genauigkeit bei der Erkennung von Kollisionen oder Bewegungen, da der taktile Sensor nicht durch äußere Einflüsse, wie zum Beispiel Vibrationen oder Stoßkräfte, beeinflusst wird.

Die starre Befestigung des taktilen Sensors kann durch verschiedene Methoden erreicht werden, wie beispielsweise Schrauben, Nieten oder Kleben. Der taktile Sensor kann auch in der ersten Komponente integriert sein oder über geeignete Adapter und Halterungen an dieser befestigt sein.

Gemäß zumindest einer Ausführungsform weist die Kollisionserkennungsvorrichtung wenigstens ein Übertragungselement auf, das in dem Innenraum angeordnet ist und einen ersten Bereich aufweist, der mit der ersten Verkleidungskomponente verbunden ist. Das wenigstens eine Übertragungselement weist einen zweiten Bereich auf, der mit der zweiten Verkleidungskomponente verbunden ist. Das wenigstens eine Übertragungselement ist derart ausgeformt, dass die erste Bewegung des Teils der zweiten Verkleidungskomponente relativ zu der ersten Verkleidungskomponente zu einer Verbiegung des wenigstens einen Übertragungselements führt, durch welche der zweite Bereich relativ zu der ersten Verkleidungskomponente bewegt wird, insbesondere relativ zu dem ersten Bereich des wenigstens einen Übertragungselements und/oder relativ zu dem ersten taktilen Sensor, und dabei den ersten taktilen Sensor betätigt. Beispielsweise ist der erste taktile Sensor im ersten Bereich an dem Übertragungselement befestigt, insbesondere starr befestigt.

Diese Konstruktion hat den Vorteil, dass die Bewegungsamplitude der zweiten Verkleidungskomponente nicht gleich der Bewegungsamplitude des ersten taktilen Sensors, insbesondere des ersten Mikroschalters, sein muss. Durch das wenigstens eine Übertragungselement kann also eine Untersetzung der Bewegung erreicht werden, sodass durch die Ausgestaltung des wenigstens einen Übertragungselements die Sensitivität der Kollisionsdetektion eingestellt beziehungsweise festgelegt werden kann.

Das wenigstens eine Übertragungselement kann beispielsweise als eines oder mehrere Bleche, Blattfedern oder Hebel ausgeformt sein, um eine geeignete Übertragung der Bewegung zu erreichen.

Gemäß zumindest einer Ausführungsform ist die erste Bewegung einer Bewegung des Teils der zweiten Verkleidungskomponente relativ zu der ersten Verkleidungskomponente, die entlang einer vorgegebenen ersten Richtung mindestens eine vorgegebene erste Mindeststrecke überstreicht.

Diese definierte Bewegung ermöglicht es, dass die Kollisionserkennungsvorrichtung gezielt auf bestimmte Bewegungen oder Kollisionen reagieren kann. Durch die Vorgabe der ersten Mindeststrecke, in manchen Ausführungsformen in Kombination mit dem Übertragungselement, wird sichergestellt, dass nur Bewegungen detektiert werden, die tatsächlich relevant sind und nicht bereits kleine Schwankungen oder Vibrationen, die beispielsweise durch externe Einflüsse verursacht werden.

Die erste Richtung und die erste Mindeststrecke können je nach Anwendung unterschiedlich definiert werden. So kann beispielsweise eine Bewegung senkrecht oder im Wesentlichen senkrecht zu einer Außenoberfläche der zweiten Verkleidungskomponente als relevant definiert werden. Auch die Länge der ersten Mindeststrecke kann entsprechend angepasst werden, um unterschiedliche effektive Schwellenwerte zu definieren. Insbesondere können die erste Richtung und die Mindeststrecke so gewählt werden, dass sie der typischen Art von Kollisionen oder Bewegungen entsprechen, die in dem entsprechenden Anwendungsfall erwartet werden. Insbesondere kann die erste Mindeststrecke derart an den Auslöseweg des ersten taktilen Sensors angepasst werden, dass in der konkreten Ausgestaltung der Verkleidungsanordnung eine Betätigung des ersten taktilen Sensors sichergestellt wird, falls die erste Bewegung in der ersten Richtung mindestens die erste Mindeststrecke überstreicht.

Gemäß zumindest einer Ausführungsform weist die Kollisionserkennungsvorrichtung einen zweiten taktilen Sensor auf, der in dem Innenraum angeordnet und dazu eingerichtet ist, bei einer zweiten Bewegung des Teils der zweiten Verkleidungskomponente oder eines weiteren Teils der zweiten Verkleidungskomponente relativ zu der ersten Verkleidungskomponente betätigt, insbesondere geschlossen, zu werden. Die zweite Bewegung einer Bewegung des Teils oder weiteren Teils der zweiten Verkleidungskomponente relativ zu der ersten Verkleidungskomponente ist, die entlang einer vorgegebenen zweiten Richtung, die von der ersten Richtung insbesondere verschieden ist, mindestens eine vorgegebene zweite Mindeststrecke überstreicht. Die Auswerteschaltung ist dazu eingerichtet, ein zweites Warnsignal zu erzeugen, wenn der zweite taktile Sensor betätigt wurde.

Dies ermöglicht es, unterschiedliche Arten von Bewegungen oder Kollisionen zu erkennen und gegebenenfalls unterschiedlich zu behandeln. Alternativ oder zusätzlich können verschiedene Teile der zweiten Verkleidungskomponente unabhängig voneinander überwacht werden. Durch die Verwendung mehrerer taktiler Sensoren und unterschiedlicher Bewegungsrichtungen kann die Kollisionserkennung flexibler und besser an die Anforderungen der Anwendung angepasst werden.

Die obigen Ausführungen zu der ersten Bewegung, der ersten Bewegungsrichtung und der ersten Mindeststrecke gelten analog für die zweite Bewegung, die zweite Bewegungsrichtung und die zweite Mindeststrecke.

Gemäß zumindest einer Ausführungsform ist der zweite taktile Sensor als Mikroschalter, insbesondere als zweiter Mikroschalter, ausgestaltet.

Gemäß zumindest einer Ausführungsform, besteht die erste Verkleidungskomponente aus einem ersten Material und die zweite Verkleidungskomponente besteht aus einem zweiten Material, wobei das zweite Material identisch zu dem ersten Material ist oder wobei eine Materialkenngröße, welche eine mechanische Verformbarkeit betrifft, für das erste Material einen ersten Wert hat und für das zweite Material einen zweiten Wert hat, der von dem ersten Wert höchstens um einen vorgegebenen Toleranzwert abweicht.

Die Materialkenngröße kann beispielsweise einem E-Modul, einer Shore-Härte, einem Kompressionsmodul oder einer Poissonzahl des jeweiligen Materials sein oder die Materialkenngröße kann von einer oder mehrerer dieser Größen abhängen.

Solche Ausführungsformen haben beispielsweise den Vorteil, dass die Integration der beiden Verkleidungskomponenten vereinfacht wird. Aufgrund der ähnlichen mechanischen Eigenschaften und somit ähnlichem Verhalten bei Belastungen oder Temperatureinflüssen können eine verbesserte Stabilität und Langlebigkeit der Verkleidungsanordnung insgesamt erreicht werden. Beide Verkleidungskomponenten werden sich aufgrund der identischen oder ähnlichen Materialien im Laufe der Lebensdauer der Verkleidungsanordnung in ähnlicher Weise verhalten. So kann beispielsweise Materialschwund oder Verformung bei beiden Komponenten ähnlich ausfallen und dadurch die Funktion der Verkleidungsanordnung über einen längeren Zeitraum erhalten bleiben. In diesem Zusammenhang kann unter mechanischer Verformbarkeit beispielsweise auch das Verhalten des Materials unter Belastung oder Temperatureinflüssen verstanden werden.

Das erste Material und das zweite Material können in manchen Ausführungsformen beispielsweise jeweils ein Hartschaummaterial und/oder ein Polyurethanschaum sein. Das erste Material und das zweite Material können in manchen Ausführungsformen beispielsweise jeweils ein thermoplastischer Kunststoff und/oder ein Acrylnitril-Butadien-Styrol-Copolymer, ABS, sein.

Diese Materialien sind besonders geeignet für Verkleidungsanordnungen, da sie eine hohe Formbeständigkeit aufweisen und dennoch flexibel genug sind gewünschte Geometrien zu erzielen.

Die Verwendung von Hartschaummaterial oder Polyurethanschaum als Material für die erste Verkleidungskomponente und die zweite Verkleidungskomponente bietet zudem den Vorteil einer besonders guten Verarbeitbarkeit sowie einer hohen Lebensdauer durch ihre gute Beständigkeit gegenüber externen Einflüssen.

Bei der Verwendung von thermoplastischem Kunststoff oder ABS als Material für die erste Verkleidungskomponente und die zweite Verkleidungskomponente ist deren besonders hohe Festigkeit von Vorteil. Diese Materialien sind auch leicht zu reinigen und weisen eine gute Chemikalienbeständigkeit auf.

Gemäß zumindest einer Ausführungsform weist die erste Verkleidungskomponente eine höhere Materialstärke auf als die zweite Verkleidungskomponente.

Durch diese Ausgestaltung wird erreicht, dass die erste Verkleidungskomponente eine erhöhte Stabilität und Steifigkeit bietet und die zweite Verkleidungskomponente leichter verformbar ist. Dadurch kann die Verkleidungsanordnung besonders stabil und sicher an der beweglichen Komponente befestigt werden, wobei dennoch eine genaue Detektion von Kollisionen möglich ist.

Insbesondere können die erste Verkleidungskomponente und die zweite Verkleidungskomponente jeweils als Hartschalenkomponente ausgestaltet sein.

Gemäß zumindest einer Ausführungsform sind die erste Verkleidungskomponente und die zweite Verkleidungskomponente mittels einer elastischen Verbindungskomponente miteinander verbunden.

Diese elastische Verbindungskomponente ermöglicht eine flexible Verbindung zwischen den beiden Verkleidungskomponenten. Die elastische Verbindungskomponente kann beispielsweise aus einem flexiblen Klebstoff oder einem Silikon oder einem Elastomer bestehen. Diese Materialien bieten den Vorteil einer hohen Flexibilität und einer guten Haftung auf den Verkleidungskomponenten.

Durch die Verwendung der elastischen Verbindungskomponente kann eine bessere Passgenauigkeit zwischen den Verkleidungskomponenten erreicht werden. Dadurch wird die Montage erleichtert und die Lebensdauer der Verkleidungsanordnung verlängert. Des Weiteren wird durch die elastische Verbindungskomponente eine Bewegung der zweiten Verkleidungskomponente relativ zu der ersten Verkleidungskomponente und die entsprechende Detektion der Kollision ohne mechanische Deformation oder mit geringerer Deformation der zweiten Verkleidungskomponente ermöglicht, sodass das Risiko für Beschädigungen der zweiten Verkleidungskomponente reduziert wird. Zudem wird das Risiko dafür reduziert, dass die Verbindung zwischen der ersten Verkleidungskomponente und der zweiten Verkleidungskomponente bei einer Kollision Schaden nimmt.

Gemäß zumindest einer Ausführungsform ist in dem Innenraum eine Strahlungsquelle, beispielsweise eine Röntgenröhre, für das medizinische Gerät zur Erzeugung einer ionisierenden Strahlung oder ein Detektor, beispielsweise ein Röntgendetektor, für das medizinische Gerät zur Detektion der ionisierenden Strahlung angeordnet.

Beispielsweise kann das medizinische Gerät einem C-Arm-Röntgenbildgebungsgerät, auch als C-Bogen-Röntgenbildgebungsgerät oder C-Arm Gerät oder C-Bogen Gerät bezeichnet, entsprechen und die bewegliche Komponente einem C-Arm, auch als C-Bogen bezeichnet, des medizinischen Geräts.

Die Erfindung ist in solchen Ausführungsformen besonders vorteilhaft, da zum einen bei Bildgebungsverfahren mittels ionisierender Strahlung mitunter Bewegungen der Strahlungsquelle und/oder des Detektors entsprechend komplexen Trajektorien und/oder hoher Bewegungsgeschwindigkeit erforderlich sind. Hier ist eine zuverlässige Kollisionserkennung daher von besonders hoher Bedeutung.

Gemäß einem weiteren Aspekt der Erfindung wird eine Abschalteinrichtung für ein medizinisches Gerät angegeben. Die Abschalteinrichtung weist eine erfindungsgemäße Verkleidungsanordnung auf sowie eine Steuereinheit, die dazu eingerichtet ist, abhängig von dem ersten Warnsignal ein Steuersignal zur Deaktivierung einer Bewegung der beweglichen Komponente zu erzeugen.

Die Steuereinheit beispielsweise kann Teil desselben Datenverarbeitungssystems sein wie die Auswerteschaltung oder eines weiteren Datenverarbeitungssystems. Die Steuereinheit kann auch die Auswerteschaltung beinhalten oder umgekehrt.

Das weitere Datenverarbeitungssystem, sofern vorgesehen, und/oder die Steuereinheit kann vollständig oder teilweise in dem Innenraum angeordnet sein oder dazu ausgebildet sein außerhalb des Innenraums, beispielsweise an der Verkleidungsanordnung oder in oder an einer anderen Stelle des medizinischen Geräts, angeordnet zu werden.

Insbesondere ist die Steuereinheit dazu eingerichtet, das Steuersignal zur Deaktivierung der Bewegung der beweglichen Komponente zu erzeugen, wenn durch das erste Warnsignal angezeigt wird, dass der erste taktile Sensor betätigt wurde.

Das medizinische Gerät weist insbesondere einen Antrieb auf, der dazu eingerichtet ist, die Bewegung der beweglichen Komponente zu verursachen. Insbesondere weist der Antrieb dazu einen oder mehrere Aktuatoren, beispielsweise Motoren, auf. Das Steuersignal kann von der Steuereinheit insbesondere an den Antrieb übermittelt werden um den Antrieb, beispielsweise den einen oder die mehreren Aktuatoren, anzusteuern, sodass die Bewegung der beweglichen Komponente nicht mehr durchgeführt werden kann oder eingestellt wird, also deaktiviert wird. Die Bewegung der beweglichen Komponente kann dann beispielsweise nur manuell wieder aktiviert werden.

In entsprechenden Ausführungsformen kann die Steuereinheit dazu eingerichtet sein, abhängig von dem zweiten Warnsignal ein Steuersignal zur Deaktivierung der Bewegung der beweglichen Komponente zu erzeugen, insbesondere abhängig von dem ersten Warnsignal und dem zweiten Warnsignal. Insbesondere ist die Steuereinheit dann dazu eingerichtet, das Steuersignal zur Deaktivierung der Bewegung der beweglichen Komponente zu erzeugen, wenn durch das erste Warnsignal angezeigt wird, dass der erste taktile Sensor betätigt wurde, oder wenn durch das zweite Warnsignal angezeigt wird, dass der zweite taktile Sensor betätigt wurde.

Weitere Ausführungsformen der erfindungsgemäßen Abschalteinrichtung folgen unmittelbar aus den verschiedenen Ausgestaltungen der erfindungsgemäßen Verkleidungsanordnung und umgekehrt. Insbesondere lassen sich einzelne Merkmale und entsprechende Erläuterungen sowie Vorteile bezüglich der verschiedenen Ausführungsformen zu der erfindungsgemäßen Verkleidungsanordnung analog auf entsprechende Ausführungsformen der erfindungsgemäßen Abschalteinrichtung übertragen.

Gemäß einem weiteren Aspekt der Erfindung wird ein medizinisches Gerät angegeben, welches eine bewegliche Komponente aufweist. Das medizinische Gerät weist eine erfindungsgemäße Verkleidungsanordnung auf, wobei die erste Verkleidungskomponente starr an der beweglichen Komponente befestigt ist. Beispielsweise weist das medizinische Gerät eine erfindungsgemäße Abschalteinrichtung auf, wobei die erste Verkleidungskomponente starr an der beweglichen Komponente befestigt ist.

In manchen Ausführungsformen ist das medizinische Gerät als mobiles, beispielsweise selbstfahrendes oder autonom fahrendes, Bildgebungsgerät ausgestaltet und/oder als C-Bogen Gerät, wobei die bewegliche Komponente einem C-Bogen des C-Bogen Geräts entspricht. Das mobile Bildgebungsgerät kann beispielsweise einem mobilen C-Bogen Gerät entsprechen.

In manchen Ausführungsformen ist das medizinische Gerät als selbstfahrendes medizinisches Geräte und/oder als Roboter für endovaskuläre oder sonstige chirurgische Eingriffe ausgebildet.

Weitere Ausführungsformen des erfindungsgemäßen medizinischen Geräts folgen unmittelbar aus den verschiedenen Ausgestaltungen der erfindungsgemäßen Verkleidungsanordnung und umgekehrt. Insbesondere lassen sich einzelne Merkmale und entsprechende Erläuterungen sowie Vorteile bezüglich der verschiedenen Ausführungsformen zu der erfindungsgemäßen Verkleidungsanordnung analog auf entsprechende Ausführungsformen des erfindungsgemäßen medizinischen Geräts übertragen.

Weitere Merkmale und Merkmalskombinationen der Erfindung ergeben sich aus den Figuren und deren Beschreibung sowie aus den Ansprüchen. Insbesondere müssen weitere Ausführungsformen der Erfindung nicht unbedingt alle Merkmale eines der Ansprüche enthalten. Weitere Ausführungsformen der Erfindungen können Merkmale oder Merkmalskombinationen aufweisen, die nicht in den Ansprüchen genannt sind.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen
- FIG 1: eine schematische Darstellung einer beispielhaften Ausführungsform eines erfindungsgemäßen medizinischen Geräts;
- FIG 2: eine schematische Schnittdarstellung einer beispielhaften Ausführungsform einer erfindungsgemäßen Verkleidungsanordnung;
- FIG 3: eine schematische Darstellung einer weiteren beispielhaften Ausführungsform einer erfindungsgemäßen Verkleidungsanordnung;
- FIG 4: eine schematische Darstellung von Details einer weiteren beispielhaften Ausführungsform einer erfindungsgemäßen Verkleidungsanordnung;
- FIG 5: eine schematische Schnittdarstellung einer weiteren beispielhaften Ausführungsform einer erfindungsgemäßen Verkleidungsanordnung; und
- FIG 6: eine schematische Schnittdarstellung einer weiteren beispielhaften Ausführungsform einer erfindungsgemäßen Verkleidungsanordnung.

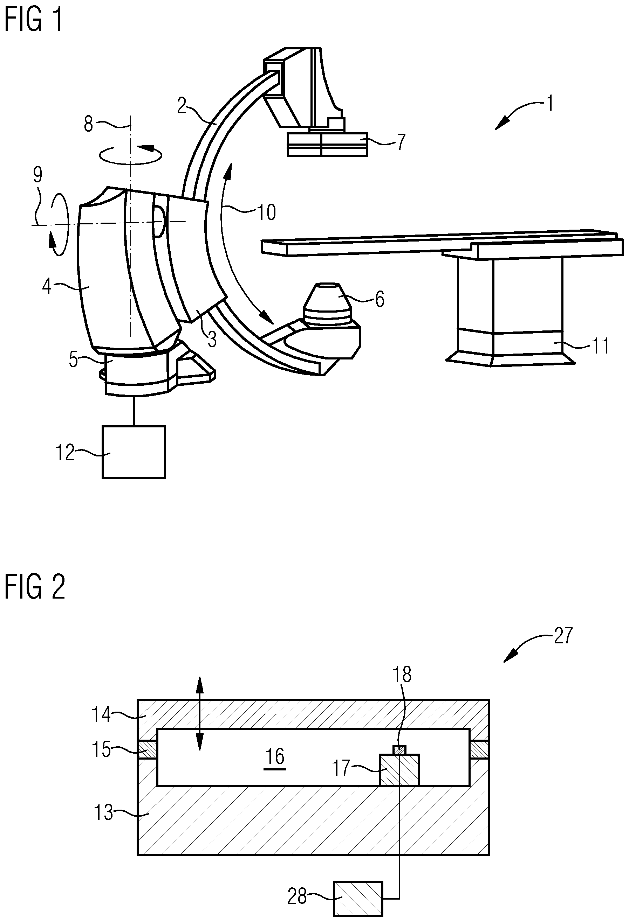

In FIG 1 ist eine beispielhafte Ausführungsform eines erfindungsgemäßen medizinischen Geräts 1 schematisch dargestellt.

Das medizinische Gerät 1 ist exemplarisch als medizinisches Bildgebungsgerät dargestellt, insbesondere als C-Arm-Röntgengerät mit einer Röntgenquelleneinheit 6, die eine Röntgenröhre beinhaltet, und einer Detektoreinheit 7, die einen Röntgendetektor beinhaltet. Ein C-Arm 2 des C-Arm-Röntgengeräts, an dem die Detektoreinheit 7 und die Röntgenquelleneinheit 6 befestigt sind, stellt eine bewegliche Komponente dar. Ferner ist eine Patientenliege 11 dargestellt. Die folgenden Erläuterungen lassen sich jedoch analog auf andere medizinische Geräte 1 mit einer beweglichen Komponente übertragen.

Das medizinische Gerät 1 weist eine erfindungsgemäße Verkleidungsanordnung 27 (siehe FIG 2 bis FIG 6) auf. Beispielsweise können die Detektoreinheit 7 und/oder die Röntgenquelleneinheit 6 einer erfindungsgemäßen Verkleidungsanordnung 27 entsprechen oder eine solche aufweisen.

Der C-Arm 2, und damit die Verkleidungsanordnung 27, kann beispielsweise in verschiedener Weise bewegt werden, insbesondere angesteuert durch eine Steuereinheit 12 des C-Arm-Röntgengeräts. Das C-Arm-Röntgengerät kann beispielsweise ein Stativ 5 aufweisen, das stationär an einem Gebäude befestigt ist. Bei mobilen C-Arm-Röntgengeräten kann das Stativ 5 auch fahrbar sein. Ein Aufbau 4 des C-Arm-Röntgengeräts ist beispielsweise drehbar um eine Drehachse 8 an dem Stativ 5 befestigt und kann angesteuert durch die Steuereinheit 12 um die Drehachse 8 rotiert werden. An dem Aufbau 4 kann beispielsweise ein Schlitten 3 befestigt sein, insbesondere drehbar um eine weitere Drehachse 9. Der Schlitten 3 kann beispielsweise angesteuert durch die Steuereinheit 12 um die weitere Drehachse 9 rotiert werden. An dem Schlitten 3 ist der C-Arm 2 verschiebbar befestigt, sodass angesteuert durch die Steuereinheit 12 eine sogenannte Orbitalbewegung 10 des C-Arm 2 ermöglicht wird.

Durch die erfindungsgemäße Ausgestaltung der Verkleidungsanordnung 27 kann eine Kollision der Verkleidungsanordnung 27 mit anderen Gegenständen oder mit Personen zuverlässig detektiert werden. In Reaktion auf eine detektierte Kollision kann beispielsweise die Steuereinheit 12 eine Bewegung des C-Arms 2 beziehungsweise aller beweglichen Komponenten des C-Arm-Röntgengeräts deaktivieren.

FIG 2 zeigt eine schematische Schnittdarstellung einer beispielhaften Ausführungsform einer erfindungsgemäßen Verkleidungsanordnung 27, wie sie beispielsweise in dem medizinischen Gerät 1 der FIG 1 eingesetzt werden kann.

Die Verkleidungsanordnung 27 weist eine erste Verkleidungskomponente 13 auf, die starr an einer beweglichen Komponente 2 des medizinischen Geräts 1 befestigt werden kann oder befestigt ist, sowie eine zweite Verkleidungskomponente 14. Die erste Verkleidungskomponente 13 und die zweite Verkleidungskomponente 14 sind mittels einer elastischen Verbindungskomponente 15 miteinander verbunden und umschließen gemeinsam einen Innenraum 16. Die Verkleidungsanordnung 27 weist eine Kollisionserkennungsvorrichtung auf, die einen ersten taktilen Sensor 18 aufweist, der in dem Innenraum 16 angeordnet und dazu eingerichtet ist, bei einer ersten Bewegung eines Teils der zweiten Verkleidungskomponente 14 relativ zu der ersten Verkleidungskomponente 13 betätigt zu werden. Die Kollisionserkennungsvorrichtung weist eine Auswerteschaltung 28 auf, die dazu eingerichtet ist, ein erstes Warnsignal zu erzeugen, wenn der erste taktile Sensor 18 betätigt wurde. Beispielsweise kann die Steuereinheit 12 abhängig von dem ersten Warnsignal ein Steuersignal zur Deaktivierung einer Bewegung der beweglichen Komponente 2 erzeugen.

Der erste taktile Sensor 18 ist beispielsweise starr an der ersten Verkleidungskomponente 13 befestigt. Dabei kann der erste taktile Sensor 18 direkt an der ersten Verkleidungskomponente 13 befestigt sein oder indirekt über eine oder mehrere weitere Komponenten, in FIG 2 rein exemplarisch durch ein Podest 17 dargestellt.

Insbesondere wird der erste taktile Sensor 18, wenn sich die zweite Verkleidungskomponente 14 relativ zu der ersten Verkleidungskomponente 13 entlang einer vorgegebenen ersten Richtung, in FIG 2 beispielsweise von oben nach unten, mindestens über eine vorgegebene erste Mindeststrecke hinweg bewegt, beispielsweise durch eine Kollision, betätigt.

Die elastische Verbindungskomponente 15 kann beispielsweise ein Klebstoff, ein Elastomer oder ein Silikon sein. Die elastische Verbindungskomponente 15 kann auch als elastische Trennfuge ausgestaltet sein.

Die erste Verkleidungskomponente 13 besteht insbesondere aus einem ersten Material und die zweite Verkleidungskomponente 14 besteht insbesondere aus einem zweiten Material. In bevorzugten Ausführungsformen hat eine Materialkenngröße, welche eine mechanische Verformbarkeit betrifft, für das erste Material einen ersten Wert und für das zweite Material einen zweiten Wert, der von dem ersten Wert höchstens um einen vorgegebenen Toleranzwert abweicht. Bei der Materialkenngröße kann es sich beispielsweise um einen E-Modul, eine Shore-Härte, einen Kompressionsmodul oder eine Poissonzahl des jeweiligen Materials handeln. In besonders bevorzugten Ausführungsformen ist das erste Material identisch zu dem zweiten Material.

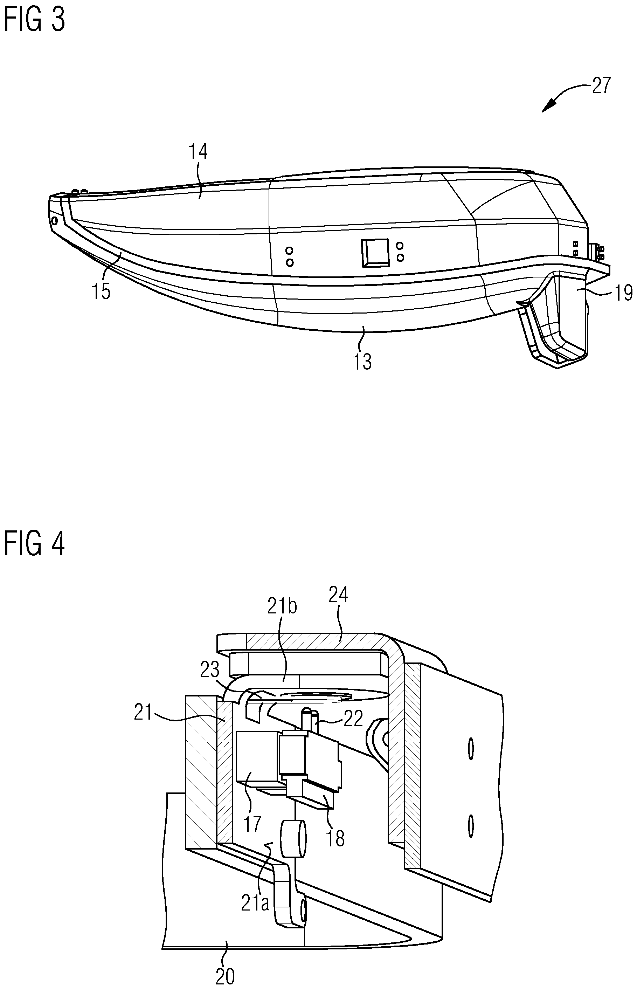

FIG 3 zeigt eine schematische Darstellung einer weiteren beispielhaften Ausführungsform einer erfindungsgemäßen Verkleidungsanordnung 27. In dieser Ausführungsform entspricht die Verkleidungsanordnung 27 beispielsweise der Röntgenquelleneinheit 6 oder der Detektoreinheit 7. Die erste Verkleidungskomponente 13 weist einen Bereich 19 zur Befestigung, insbesondere Verschraubung, der Verkleidungsanordnung 27 an dem C-Arm 2 auf.

In FIG 4 sind Details einer weiteren beispielhaften Ausführungsform einer erfindungsgemäßen Verkleidungsanordnung 27 schematisch dargestellt. Hierbei sind ein starr an der ersten Verkleidungskomponente 13 im Innenraum 16 befestigtes Bauteil 20 und ein starr an der zweiten Verkleidungskomponente 14 im Innenraum 16 befestigtes Bauteil 24 dargestellt. Bei den Bauteilen 20, 24 kann es sich beispielsweise um Bleche oder entsprechende Kunststoffbauteile handeln.

Die Kollisionserkennungsvorrichtung weist ein Übertragungselement 21 auf, das in dem Innenraum 16 angeordnet ist und einen ersten Bereich 21a aufweist, der mit der ersten Verkleidungskomponente 13, insbesondere über das Bauteil 20, verbunden ist, sowie einen zweiten Bereich 21b, der mit der zweiten Verkleidungskomponente 14, insbesondere über das Bauteil 24, verbunden ist. Das Übertragungselement 21 kann ebenfalls ein Blech oder ein entsprechendes Kunststoffbauteil sein.

Beispielsweise ist der erste taktile Sensor 18 hier als erster Mikroschalter 18 ausgestaltet. Das Übertragungselement 21 ist derart ausgeformt, dass die erste Bewegung des Teils der zweiten Verkleidungskomponente 14 relativ zu der ersten Verkleidungskomponente 13 zu einer Verbiegung des Übertragungselements 21 führt, durch welche der zweite Bereich 21 relativ zu der ersten Verkleidungskomponente 13 bewegt wird und dabei den ersten Mikroschalter 18 betätigt. Im Beispiel der FIG 4 weist der erste Mikroschalter 18 beispielsweise eine Auslöser 22 auf, der durch eine Bewegung von oben nach unten betätigt werden kann. Daher kann die Bewegung der zweiten Verkleidungskomponente 14 beispielsweise einer Bewegung von oben nach unten entsprechen. Durch entsprechende Ausformung des Bauteils 24 kann aber auch erreicht werden, dass eine Bewegung von rechts nach links zu einer Verbiegung des Übertragungselements 21 führt, durch welche der zweite Bereich 21 relativ zu der ersten Verkleidungskomponente 13 bewegt wird und dabei den ersten Mikroschalter 18 betätigt.

Es ist möglich, dass das Übertragungselement 21 direkt auf den ersten Mikroschalter 18 beziehungsweise dessen Auslöser 22 wirkt. Alternativ kann auch ein Zwischenbauteil 23, beispielsweise ein Blech, zwischen dem Übertragungselement 21 und dem Auslöser 22 angeordnet sein, beispielsweise an dem Übertragungselement 21 befestigt, welches durch die Verbiegung des Übertragungselement 21 zur Betätigung des ersten Mikroschalters 18, insbesondere des Auslösers 22, führt. Das Zwischenbauteil 23 kann auch als weiteres Übertragungselement 21 angesehen werden.

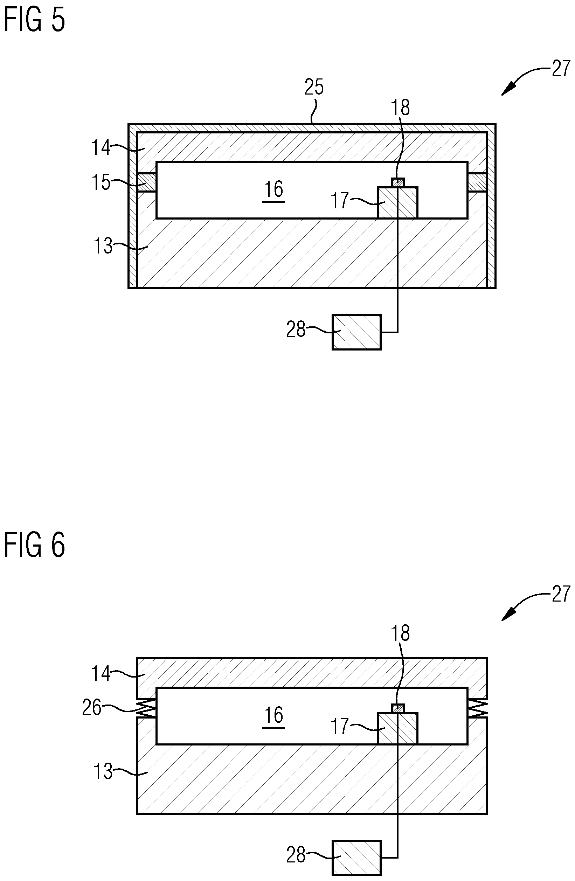

FIG 5 zeigt eine schematische Schnittdarstellung einer weiteren beispielhaften Ausführungsform einer erfindungsgemäßen Verkleidungsanordnung 27, die auf der Ausführungsform der FIG 2 basiert.

Hierbei ist eine Beschichtung 25 vorgesehen, welche die erste Verkleidungskomponente 13 wenigstens zum Teil überdeckt und/oder die zweite Verkleidungskomponente 14 wenigstens zum Teil überdeckt und/oder die Verbindungskomponente 14 wenigstens zum Teil überdeckt. Die Beschichtung 25 ist insbesondere eine elastische Beschichtung 25. Die Beschichtung 25 kann insbesondere weitere Eigenschaften wie Biokompatibilität realisieren und/oder eine antimikrobielle Wirkung aufweisen.

FIG 6 zeigt eine schematische Schnittdarstellung einer weiteren beispielhaften Ausführungsform einer erfindungsgemäßen Verkleidungsanordnung 27, die auf der Ausführungsform der FIG 2 basiert.

In dieser Ausführungsform sind die erste Verkleidungskomponente 13 und die zweite Verkleidungskomponente 14 mittels einer Verbindungskomponente 26 miteinander verbunden, deren Ausdehnung in Richtung von der ersten Verkleidungskomponente 13 zu der zweiten Verkleidungskomponente 14 variabel ist. Hierzu kann die Verbindungskomponente 26 beispielsweise "ziehharmonikaartig" ausgebildet sein.

Wie beschrieben bietet die Erfindung eine einfache und robuste Möglichkeit zur Kollisionserkennung einer beweglichen Komponente eines medizinischen Geräts, durch welche die Nachteile anderer Lösungen überwunden werden können.

In manchen Ausführungsformen sind einer oder mehrere Mikroschalter fest an der beweglichen Komponente verschraubt oder mittels Hilfsblechen befestigt.

Unabhängig vom grammatikalischen Geschlecht eines bestimmten Begriffes sind Personen mit männlicher, weiblicher oder anderer Geschlechteridentität mit umfasst.

## Patentansprüche

1. Verkleidungsanordnung (27) für ein medizinisches Gerät (1) aufweisend eine erste Verkleidungskomponente (13) zur starren Befestigung an einer beweglichen Komponente (2) des medizinischen Geräts (1), sowie eine zweite Verkleidungskomponente (14), wobei
- die erste Verkleidungskomponente (13) und die zweite Verkleidungskomponente (14) mittels einer elastischen Verbindungskomponente (15) miteinander verbunden sind und gemeinsam einen Innenraum (16) umschließen;
- die Verkleidungsanordnung (27) eine Kollisionserkennungsvorrichtung aufweist, die einen taktilen Sensor (18) aufweist, der in dem Innenraum (16) angeordnet und dazu eingerichtet ist, bei einer ersten Bewegung eines Teils der zweiten Verkleidungskomponente (14) relativ zu der ersten Verkleidungskomponente (13) betätigt zu werden;
- die Kollisionserkennungsvorrichtung eine Auswerteschaltung (28) aufweist, die dazu eingerichtet ist, ein erstes Warnsignal zu erzeugen, wenn der erste taktile Sensor (18) betätigt wurde.

2. Verkleidungsanordnung (27) nach Anspruch 1, wobei der erste taktile Sensor (18) starr an der ersten Verkleidungskomponente (13) befestigt ist.

3. Verkleidungsanordnung (27) nach einem der vorhergehenden Ansprüche, wobei
- die Kollisionserkennungsvorrichtung wenigstens ein Übertragungselement (21) aufweist, das in dem Innenraum (16) angeordnet ist und einen ersten Bereich (21a) aufweist, der mit der ersten Verkleidungskomponente (13) verbunden ist, sowie einen zweiten Bereich (21b), der mit der zweiten Verkleidungskomponente (14) verbunden ist;
- das wenigstens eine Übertragungselement (21) derart ausgeformt ist, dass die erste Bewegung des Teils der zweiten Verkleidungskomponente (14) relativ zu der ersten Verkleidungskomponente (13) zu einer Verbiegung des wenigstens einen Übertragungselements (21) führt, durch welche der zweite Bereich (21b) relativ zu der ersten Verkleidungskomponente (13) bewegt wird und dabei den ersten taktilen Sensor (18) betätigt.

4. Verkleidungsanordnung (27) nach Anspruch 3, wobei der erste taktile Sensor (18) im ersten Bereich (21a) an dem Übertragungselement (21) befestigt ist.

5. Verkleidungsanordnung (27) nach einem der vorhergehenden Ansprüche, wobei die erste Bewegung einer Bewegung des Teils der zweiten Verkleidungskomponente (14) relativ zu der ersten Verkleidungskomponente (13) ist, die entlang einer vorgegebenen ersten Richtung mindestens eine vorgegebene erste Mindeststrecke überstreicht.

6. Verkleidungsanordnung (27) nach einem der vorhergehenden Ansprüche, wobei
- die Kollisionserkennungsvorrichtung einen zweiten taktilen Sensor aufweist, der in dem Innenraum (16) angeordnet und dazu eingerichtet ist, bei einer zweiten Bewegung des Teils der zweiten Verkleidungskomponente (14) oder eines weiteren Teils der zweiten Verkleidungskomponente (14) relativ zu der ersten Verkleidungskomponente (13) betätigt zu werden;
- die zweite Bewegung einer Bewegung des Teils oder weiteren Teils der zweiten Verkleidungskomponente (14) relativ zu der ersten Verkleidungskomponente (13) ist, die entlang einer vorgegebenen zweiten Richtung mindestens eine vorgegebene zweite Mindeststrecke überstreicht; und
- die Auswerteschaltung (28) dazu eingerichtet ist, ein zweites Warnsignal zu erzeugen, wenn der zweite taktile Sensor betätigt wurde.

7. Verkleidungsanordnung (27) nach einem der vorhergehenden Ansprüche, wobei die erste Verkleidungskomponente (13) aus einem ersten Material besteht und die zweite Verkleidungskomponente (14) aus einem zweiten Material besteht, wobei das zweite Material identisch zu dem ersten Material ist oder wobei eine Materialkenngröße, welche eine mechanische Verformbarkeit betrifft, für das erste Material einen ersten Wert hat und für das zweite Material einen zweiten Wert hat, der von dem ersten Wert höchstens um einen vorgegebenen Toleranzwert abweicht.

8. Verkleidungsanordnung (27) nach Anspruch 7, wobei
- das erste Material und das zweite Material jeweils ein Hartschaummaterial und/oder ein Polyurethanschaum sind; oder
- das erste Material und das zweite Material jeweils ein thermoplastischer Kunststoff sind und/oder ein Acrylnitril-Butadien-Styrol-Copolymer.

9. Verkleidungsanordnung (27) nach einem der Ansprüche 7 oder 8, wobei die Materialkenngröße von einem E-Modul und/oder einer Shore-Härte und/oder einem Kompressionsmodul und/oder einer Poissonzahl des jeweiligen Materials abhängt.

10. Verkleidungsanordnung (27) nach einem der Ansprüche 7 bis 9, wobei erste Verkleidungskomponente (13) eine höhere Materialstärke aufweist als die zweite Verkleidungskomponente (14).

11. Verkleidungsanordnung (27) nach einem der vorhergehenden Ansprüche, wobei der erste taktile Sensor (18) als Mikroschalter ausgestaltet ist.

12. Verkleidungsanordnung (27) nach einem der vorhergehenden Ansprüche, wobei in dem Innenraum (16) eine Strahlungsquelle für das medizinische Gerät (1) zur Erzeugung einer ionisierenden Strahlung oder ein Detektor für das medizinische Gerät (1) zur Detektion der ionisierenden Strahlung angeordnet ist.

13. Abschalteinrichtung für ein medizinisches Gerät (1) aufweisend eine Verkleidungsanordnung (27) nach einem der vorhergehenden Ansprüche sowie eine Steuereinheit (12), die dazu eingerichtet ist, abhängig von dem ersten Warnsignal ein Steuersignal zur Deaktivierung einer Bewegung der beweglichen Komponente (2) zu erzeugen.

14. Medizinisches Gerät (1) aufweisend eine bewegliche Komponente (2) und eine Verkleidungsanordnung (27) nach einem der Ansprüche 1 bis 12 oder eine Abschalteinrichtung nach Anspruch 13, wobei die erste Verkleidungskomponente (13) starr an der beweglichen Komponente (2) befestigt ist.

15. Medizinisches Gerät (1) nach Anspruch 14, wobei das medizinische Gerät (1) als mobiles Bildgebungsgerät ausgestaltet ist und/oder wobei das medizinische Gerät (1) als C-Bogen Gerät ausgestaltet ist und die bewegliche Komponente (2) einem C-Bogen des C-Bogen Geräts entspricht und/oder wobei das medizinische Gerät (1) als Roboter für Roboter für endovaskuläre Eingriffe oder sonstige chirurgische Eingriffe ausgestaltet ist.
